# EUROPEAN PATENT APPLICATION

(11) **EP 1 236 738 A1**
(43) Date of publication of application: **04.09.2002**
(21) Application number: 01104633.1
(22) Date of filing: 23.02.2001
(51) Int. Cl.: C07K 14/435, C07K 14/47, C12N 15/36, C12N 15/62

(54) **Internal standard for electrophoretic and chromatographic separation process**

(71) Applicant: Jungbauer, Alois, Professor Dr., 1180 Wien (AT)
(72) Inventor: Jungbauer, Alois Herr Prof.Dr., 1210 Wien (AT); Einhauer, Adelheid Frau, 1090 Wien (AT)
(74) Representative: Meyers, Hans-Wilhelm, Dr.Dipl.-Chem.

(57) **Abstract**

A fusion protein having the amino acid sequence of a marker protein and the amino acid sequence of a second protein so selected that it yields a predeterminded mass and/or isoelectric point making the fusion protein suitable to represent a reference protein (landmark) in an analytical method as well as the method of using the fusion protein. Preferably, the fusion protein comprises a marker protein which is selected from the group consisting of Green Fluorescent Protein (GFP), BFP, and the second protein is selected from the group consisting of EIF5a, Hsp90beta, FKBp25, lysozym.

SEQUENCE LISTING

<110> Prof. Dr. Alois Jungbauer

<120> Internal standard for electrophoretic and
      chromatographic separation processes

<130> Prof. Dr. Alois Jungbauer

<140>
<141>

<160> 6

<170> PatentIn Ver. 2.1

<210> 1
<211> 720
<212> DNA
<213> Artificial Sequence

<220>
<223> Description of Artificial Sequence: fusion
      proteins

<400> 1
atggctagca aaggagaaga actcttcact ggagttgtcc caattcttgt tgaattagat 60
ggtgatgtta acggccacaa gttctctgtc agtggagagg gtgaaggtga tgcaacatac 120
ggaaaactta ccctgaagtt catctgcact actggcaaac tgcctgttcc atggccaaca 180
ctagtcacta ctctgtgcta tggtgttcaa tgcttttcaa gatacccgga tcatatgaaa 240
cggcatgact ttttcaagag tgccatgccc gaaggttatg tacaggaaag gaccatcttc 300
ttcaaagatg acggcaacta caagacacgt gctgaagtca agtttgaagg tgataccctt 360
gttaatagaa tcgagttaaa aggtattgac ttcaaggaag atggcaacat tctgggacac 420
aaattggaat acaactataa ctcacacaat gtatacatca tggcagacaa acaaaagaat 480
ggaatcaaag tgaacttcaa gacccgccac aacattgaag atggaagcgt tcaactagca 540
gaccattatc aacaaaatac tccaattggc gatggccctg tccttttacc agacaaccat 600
tacctgtcca cacaatctgc cctttcgaaa gatcccaacg aaaagagaga ccacatggtc 660
cttcttgagt ttgtaacagc tgctgggatt acacatggca tggatgaact gtacaactga 720

<210> 2
<211> 720
<212> DNA
<213> Artificial Sequence

<220>
<223> Description of Artificial Sequence: fusion
      proteins

<400> 2
atggctagca aaggagaaga actcttcact ggagttgtcc caattcttgt tgaattagat 60
ggtgatgtta acggccacaa gttctctgtc agtggagagg gtgaaggtga tgcaacatac 120
ggaaaactta ccctgaagtt catctgcact actggcaaac tgcctgttcc atggccaaca 180
ctagtcacta ctctctctca tggtgttcaa tgcttttcaa gatacccgga tcatatgaaa 240
cggcatgact ttttcaagag tgccatgccc gaaggttatg tacaggaaag gaccatcttc 300
ttcaaagatg acggcaacta caagacacgt gctgaagtca agtttgaagg tgataccctt 360
gttaatagaa tcgagttaaa aggtattgat tttaaagaag atggaaacat tcttggacac 420
aaattggaat acaactataa ctcacacaat gtatacatca tggcagacaa acaaaagaat 480
ggaatcaaag cgaacttcaa gatccgccac aacattgaag atggaagcgt tcaactagca 540
gaccattatc aacaaaatac tccaattggc gatggccctg tccttttacc agacaaccat 600
tacctgtcca cacaatctgc cctttcgaaa gatcccaacg aaaagagaga ccacatggtc 660
cttcttgagt ttgtaacagc tgctgggatt acacatggca tggatgaact atacaaataa 720

<210> 3
<211> 675
<212> DNA
<213> Artificial Sequence

<220>
<223> Description of Artificial Sequence: fusion
      proteins

<400> 3
atggcggcgg ccgttccaca gcgggcgtgg accgtggagc agctgcgcag tgagcagctg 60
cccaagaagg acattatcaa gtttctgcag gaacacggtt cagattcgtt tcttgcagaa 120
cataaattat taggaaacat taaaaatgtg gccaagacag ctaacaagga ccacttggtt 180
acagcctata accatctttt tgaaactaag cgttttaagg gtactgaaag tataagtaaa 240
gtgtctgagc aagtaaaaaa tgtgaagctt aatgaagata aacccaaaga aaccaagtct 300
gaagagaccc tggatgaggg tccaccaaaa tatactaaat ctgttctgaa aaagggagat 360
aaaaccaact ttcccaaaaa gggagatgtt gttcactgct ggtatacagg aacactacaa 420
gatgggactg tttttgatac taatattcaa acaagtgcaa agaagaagaa aaatgccaag 480
cctttaagtt ttaaggtcgg agtaggcaaa gttatcagag gatgggatga agctctcttg 540
actatgagta aaggagaaaa ggctcgactg gagattgaac cagaatgggc ttacggaaag 600
aaaggacagc ctgatgccaa aattccacca aatgcaaaac tcacttttga agtggaatta 660
gtggatattg attga 675

<210> 4
<211> 465
<212> DNA
<213> Artificial Sequence

<220>
<223> Description of Artificial Sequence: fusion
      proteins

<400> 4
atggcagatg acttggactt cgagacagga gatgcagggg cctcagccac cttcccaatg 60
cagtgctcag cattacgtaa gaatggcttt gtggtgctca aaggccggcc atgtaagatc 120
gtcgagatgt ctacttcgaa gactggcaag cacggccacg ccaaggtcca tctggttggt 180
attgacatct ttactgggaa gaaatatgaa gatatctgcc cgtcaactca taatatggat 240
gtccccaaca tcaaaaggaa tgacttccag ctgattggca tccaggatgg gtacctatca 300
ctgctccagg acagcgggga ggtacgagag gaccttcgtc tccctgaggg agaccttggc 360
aaggagattg agcagaagta cgactgtgga gaagagatcc tgatcacggt gctgtctgcc 420
atgacagagg aggcagctgt tgcaatcaag gccatggcaa aataa 465

<210> 5
<211> 2175
<212> DNA
<213> Artificial Sequence

<220>
<223> Description of Artificial Sequence: fusion
      proteins

<400> 5
atgcctgagg aagtgcacca tggagaggag gaggtggaga cttttgcctt tcaggcagaa 60
attgcccaac tcatgtccct catcatcaat accttctatt ccaacaagga gattttcctt 120
cgggagttga tctctaatgc ttctgatgcc ttggacaaga ttcgctatga gagcctgaca 180
gacccttcga agttggacag tggtaaagag ctgaaaattg acatcatccc caaccctcag 240
gaacgtaccc tgactttggt agacacaggc attggcatga ccaaagctga tctcataaat 300
aatttgggaa ccattgccaa gtctggtact aaagcattca tggaggctct tcaggctggt 360
gcagacatct ccatgattgg gcagtttggt gttggctttt attctgccta cttggtggca 420
gagaaagtgg ttgtgatcag aaagcacaac gatgatgaac agtatgcttg ggagtcttct 480
gctggaggtt ccttcactgt gcgtgctgac catggtgagc ccattggcat gggtaccaaa 540
gtgatcctcc atcttaaaga agatcagaca gagtacctag aagagaggcg ggtcaaagaa 600
gtagtgaaga agcattctca gttcataggc tatcccatca ccctttattt ggagaaggaa 660
cgagagaagg aaattagtga tgatgaggca gaggaagaga aaggtgagaa agaagaggaa 720
gataaagatg atgaagaaaa gcccaagatc gaagatgtgg gttcagatga ggaggatgac 780
agcggtaagg ataagaagaa gaaaactaag aagatcaaag agaaatacat tgatcaggaa 840
gaactaaaca agaccaagcc tatttggacc agaaaccctg atgacatcac ccaagaggag 900
tatggagaat tctacaagag cctcactaat gactgggaag accacttggc agtcaagcac 960
ttttctgtag aaggtcagtt ggaattcagg gcattgctat ttattcctcg tcgggctccc 1020
tttgaccttt ttgagaacaa gaagaaaaag aacaacatca aactctatgt ccgccgtgtg 1080
ttcatcatgg acagctgtga tgagttgata ccagagtatc tcaattttat ccgtggtgtg 1140
gttgactctg aggatctgcc cctgaacatc tcccgagaaa tgctccagca gagcaaaatc 1200
ttgaaagtca ttcgcaaaaa cattgttaag aagtgccttg agctcttctc tgagctggca 1260
gaagacaagg agaattacaa gaaattctat gaggcattct ctaaaaatct caagcttgga 1320
atccacgaag actccactaa ccgccgccgc ctgtctgagc tgctgcgcta tcatacctcc 1380
cagtctggag atgagatgac atctctgtca gagtatgttt ctcgcatgaa ggagacacag 1440
aagtccatct attacatcac tggtgagagc aaagagcagg tggccaactc agcttttgtg 1500
gagcgagtgc ggaaacgggg cttcgaggtg gtatatatga ccgagcccat tgacgagtac 1560

tgtgtgcagc agctcaagga atttgatggg aagagcctgg tctcagttac caaggagggt 1620
ctggagctgc ctgaggatga ggaggagaag aagaagatgg aagagagcaa ggcaaagttt 1680
gagaacctct gcaagctcat gaaagaaatc ttagataaga aggttgagaa ggtgacaatc 1740
tccaatagac ttgtgtcttc accttgctgc attgtgacca gcacctacgg ctggacagcc 1800
aatatggagc ggatcatgaa agcccaggca cttcgggaca actccaccat gggctatatg 1860
atggccaaaa agcacctgga gatcaaccct gaccacccca ttgtggagac gctgcggcag 1920
aaggctgagg ccgacaagaa tgataaggca gttaaggacc tggtggtgct gctgtttgaa 1980
accgccctgc tatcttctgg cttttccctt gaggatcccc agacccactc caaccgcatc 2040
tatcgcatga tcaagctagg tctaggtatt gatgaagatg aagtggcagc agaggaaccc 2100
aatgctgcag ttcctgatga gatcccccct ctcgagggcg atgaggatgc gtctcgcatg 2160
gaagaagtcg attag 2175

<210> 6
<211> 390
<212> DNA
<213> Artificial Sequence

<220>
<223> Description of Artificial Sequence: fusion
      proteins

<400> 6
aaagtctttg gacgatgtga gctggcagcg gctatgaagc gtcacggact tgataactat 60
cggggataca gcctgggaaa ctgggtgtgt gccgcaaaat tcgagagtaa cttcaacacc 120
caggctacaa accgtaacac cgatgggagt accgactacg gaatcctaca gatcaacagc 180
cgctggtggt gcaacgatgg caggacccca ggctccagga acctgtgcaa catcccgtgc 240
tcagccctgc tgagctcaga cataacagcg agcgtgaact gcgcgaagaa gatcgtcagc 300
gatggaaacg gcatgaacgc gtgggtcgcc tggcgcaacc gctgcaaggg caccgacgtc 360
caggcgtgga tcagaggctg ccggctgtga 390

## Description

The present invention is related with a fusion protein having the amino acid sequence of a marker protein and the amino acid sequence of a second protein, a method for manufacturing the fusion protein of the invention as well as a method of using the fusion protein of the invention.

A procedure and reference proteins have been developed to enable unequivocal identification of protein samples of interest. A special production procedure has been utilized to generate extremely homogenous proteins leading to either very defined spots in 2D-electrophoresis, sharp bands in 1D-electrophoresis and chromatography.

2D electrophoresis represents the most powerful analytical tool for separation and characterization of proteins. Visualization of the 2D protein spot patterns is achieved usually by staining the proteins while present in the 2Dgel with a dye, which is most commonly Coomassie brilliant blue R-250, or by deposition of metallic silver. Alternatively, proteins which have been radiolabeled before analysis in vivo or in vitro can be detected in the 2D gel by autoradiography. This technique involves the handling of the second-dimensional gel and may require a period of several hours more after the end of the electrophoresis before a spot pattern becomes visible. Additional limitations of the method are low reproducibility and lack of an internal standard for determination of electrophoretic mobility, when complex samples are analyzed.

Conventional protein staining with dyes such as Coomassie blue or silver nitrate is rather unspecific. Immunological methods are highly specific but fail when cross-reactions take place. Both methods must be combined to unequivocally identify a protein spot. Due to electrochemical processes such as electroendoosmosis, due to diffusion resistances and due to non-reproducibility of gel formation, the migration pattern of identical samples differs from gel to gel. Therefore characteristic spots have been used as so called landmarks to collate protein location in a gel. Through the landmarks a two dimensional grid is constructed enabling the assignment of the unknown protein. Shape and position of the landmark can be effected by the sample preparation and in many cases the landmark and the protein of interest are widely remoted from each other. Even so an unequivocal assignment of the protein is still possible.

By nature proteins are heterogeneous. Several amino acids are prone to undergo chemical modifications. Currently it is not clear if modifications such as deamidation for example take already place during translation. Environmental changes may accelerate such a process. Posttranslational modifications of side chains lead to further heterogeneity of a protein. Numerous modifications have been reported. Not all of them are of great biological relevance. Glycosylation and phosphorylation have been studied to a great extend. It has been shown that there are big varieties in the modification pattern with regard to host cells and expression strategies.

It is an object of the invention to eliminate as far as possible the shortcomings and drawbacks of the methods and standard proteins of prior art.

The underlying technical problem is solved by a fusion protein having the amino acid sequence of a marker protein and the amino acid sequence of a second protein so selected that it yields a predeterminded mass and/or isoelectric point making the fusion protein suitable to represent a reference protein (landmark) in an analytical method.
- Fig. 1: shows a schematic grid obtained by fusion proteins of the invention used in an electrophoresis.
- Fig. 2: shows a schematic map of expression plasmid YepB1.

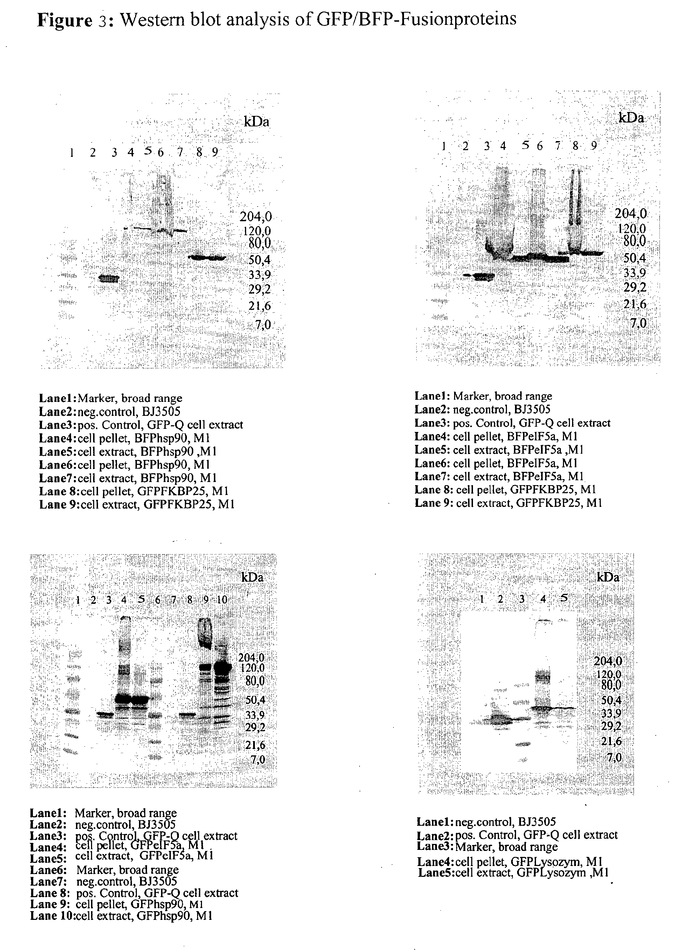

- Fig. 3: shows a western blot analysis of GFP/BFP fusion proteins.

The fusion protein of the invention is preferably expressed in S. cerevisiae. A variety of host-cells is used for over expression of recombinant proteins. Due to several reasons intracellular expression in yeast is advantageous. Expression in prokaryotic cells such as Escherichia coli often led to an unfolded or incorrectly folded protein, especially when disulfide bonds are part of the protein structure. Endotoxin formation of recombinant proteins expressed in E.coli represents a potential immunogenicity problem when produced for biopharmaceutical applications. Yeast such as Saccharomyces cerevisiae has less tendency for inclusion body formation and lacks endotoxin formation. As a result, for biotechnological purposes Saccharomyces cerevisiae obtained GRAS (generally regarded as safe) status. Additional major advantages of yeast are its fast cultivation, simple manipulation and handling compared to animal cells and low nutrition requirements. Yeast lacks complex posttranslational modifications such as N-glycosylations for intracellularly expressed proteins as demonstrated by Schuster et. al 2000. Therefore homogenous products can be expected.

The fusion proteins of the invention are preferably those in which the marker protein is selected from the group consisting of Green Fluorescent Protein (GFP), Blue Fluorescent Protein (BFP), and the second protein is selected from the group of proteins having high solubility, stability and expression level in yeast such as EIF5a, Hsp90beta, FKBp25, lysozym.

In particular preferred are the following fusion proteins having the DNA sequences:
Seg. ID. No. 1 to Seq, ID. No. 6

Defined proteins have been developed to simplify characterization of proteins in high resolution methods such as 2D-electrophoresis, CE or hyphenated separation methods. Special emphasis was put on the fact that glycosylation would dramatically effect the heterogeneity of a protein leading to tremendous changes of molecular mass and electrophoretic properties. As a consequence an expression strategy was chosen to circumvent this part of posttranslational modifications. Proteins were expressed in yeast intracellularly.

The invention provides also a method of using at least one of the fusion proteins of the invention as a landmark in a multi-stage process for detection of proteins which discriminates different proteins in terms of size, electrophoretic mobility and isoelectric point.

According to the method of the invention a fusion protein is mixed as a standard reference into an analytical sample applied to a separation medium, and after separation is detected by an appropriate property such as fluorescence or chemiluminescence, staining or blotting and detection with affinity ligands such as labelled antibodies, enzyme conjugated antibodies. Preferably GFP-fusion proteins as descried - produced by S. cerevisiae are mixed to the protein sample of interest and then a 2D electrophoresis is performed using standard protocols as described by Westermaier e.g..

After the electrophoretic separation is completed the gel is illuminated. Due to a CCD camera linked data acquisition system such as the Lumi Imager, Roche, a grid of landmarks becomes visible. The position of the individual landmarks is stored and then the second staining is performed to stain the sample proteins. Again the spot pattern is captured by the digital camera and then by overlay of the two pictures an extract collation of the unknown spots can be performed.

An analytical kit comprising at least one fusion protein of the invention is also subject of the present invention. Typically the analytical kit contains also buffer systems or prefabricated gels which can be used in the analytical separations for which the fusion proteins of the present invention are used.

To enable a multi-stage detection procedure, fusion proteins were developed in a way which allows detection of proteins with at least two independent detection principles. To this end, according to the invention it is preferred to employ at least two fusion proteins of the invention which proteins have different marker proteins and/or different second proteins.

As fusion partner a fluorescent or chemiluminescent protein tag was chosen. This tag represents the first detection principle and allows immediate visualization and construction of a grid of landmarks or other reference proteins by excitation at the corresponding wavelength.

The method of the invention is preferably employed in electrophoretical methods. To develop an optimal grid of reference proteins or landmarks, fusion partners to the fluorescent or chemiluminescent tag must be selected according to the expected molecular mass and isoelectric point of the protein sample of interest. The purified fusion proteins are mixed to the sample of interest and applied to a high Resolution separation method of choice, for example 2D-electrophoresis, CE Capillary zone electrophoresis, isotachophoresis, capillary electrochromatography, chromatofocusing and high performance ion-exchange chromatography. The reference proteins are easily detected by fluorescent, chemiluminescent of immunochemical detection procedures, while the other ones can be detected by less selective methods, such as staining or UV adsorption for example. Thus, false positive results caused by cross reactions of the antibody at Westem blot analysis for example can be circumvented.

To further optimize the allocation of the sample, recombinant proteins and fragments can be added to the sample of interest. These proteins are either tagged with an alternative fluorescent or chemiluminescent protein or a tag enabling immunochemical detection. Again these proteins must be expressed in a manner to be well defined.

The invention is further illustrated by the following non limiting examples.

### Example

### Design of an internal standard for 2D-electrophoresis

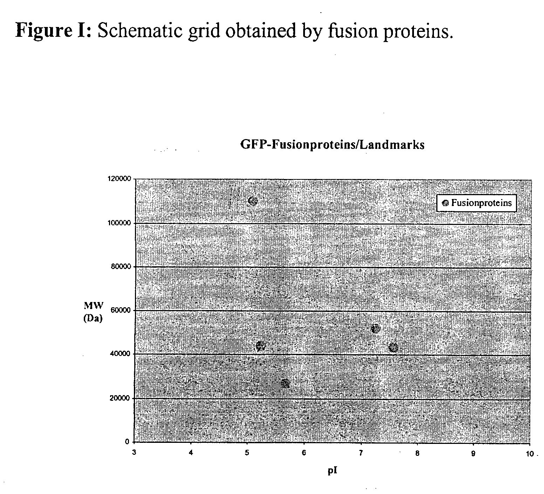

Fusion proteins are constructed as described in example 1 in a way to cover the interesting area of a 2D electrophoresis gel regarding isoelectric point and molecular mass. By fusing a fluorescent protein to a number of well-defined selected proteins, the isoelectric point and the molecular mass can be designed in a respective manner in order to obtain a grid which can act as an internal standard. An example for such a grid is shown in Figure 1.

By the addition of another set of fusion proteins to the internal standards for the grid, a second staining procedure can be performed simultaneously. After blotting such a 2D-gel onto a membrane, for example nitrocellulose, the gridstandards can be visualized by fluorescence, the second set of standard proteins can be detected immunologically by using an antifusion antibody which is labeled with a fluorescent label differing in the emission spectrum compared to the first standard set. These samples can be detected with specific antibodies. If blotting should be avoided a conventionel staining procedure also can be performed. The landmarks can be illuminated and the picture is recorded by photoimaging. Subsequently a conventional staining procedure can be applied and again the picture is recorded. Both images are superimposed. As a result the grid can be applied to the protein spots on the stained gel and an unequivocal identification is possible.

Great demands are made on the construction of the landmarks since there exists no internal standard for 2D-electrophoresis to date which comprises unequivocal identification of protein spots in both, 2D-gels and Westem blot membranes. Green/Blue fluorescent protein has to be N-terminally fused to an appropriate partner. Fusion partners have to meet several criteria such as high expression levels in yeast, homogenous translation products as well as an appropriate molecular mass and isoelectric point. Selected fusion partners with their corresponding isoelectric points and molecular masses are listed in Table 1. The resulting isoelectric points and molecular masses of the resulting GFP/BFP fusion proteins are listed in Table 1.

**Table 1:**

| List of selected GFP fusion partners. | | |
|---|---|---|
| Protein | pI | MW (Da) |
| GFP/BFP | 5.67 | 26,886.3 |
| EIF5a | 4.81 | 16,983.0 |
| Hsp90beta | 4.97 | 83,163.0 |
| FKBP25 | 9.29 | 25,176.8 |
| Lysozym | 11.0 | 14,3889.0 |

### Vector construction

An episomal yeast/Escherichia coli shuttle vector was constructed for the over expression of the landmarks in Saccharomyces cerevisiae as described by Schuster et. al.. The vector contains two origins of replication (ori), the procaryotic Col E1 ori as well as the eucaryotic 2µ ori and can therefore replicate autonomously in both organisms. Resistance to ampicillin (amp^{r}) encoded by the β-lactamase gene in the plasmid and tryptophan prototrophy of the host strain BJ3505 permit selection of positive transformants in both, E.coli and S.cerevisiae. The expression cassette comprises the copper inducible metallothionein (Cupl) promoter, the sequence for the ubi4 (ubiquitin) gene inducing protein over expression, the FLAG-tag (an octapeptide coding for the sequence DYKDDDDK as described by Hopp et. al. 1988) which facilitates immunological detection and purification of the expressed protein, a multiple cloning site for in-frame insertion of the genes as Sall/Notl fragments as well as a CYCI region, facilitating transcriptional termination in BJ3505. During maturation of the expressed fusion protein ubiquitin will be removed by proteolytic enzymes resulting in an N-terminally FLAG-tagged protein.

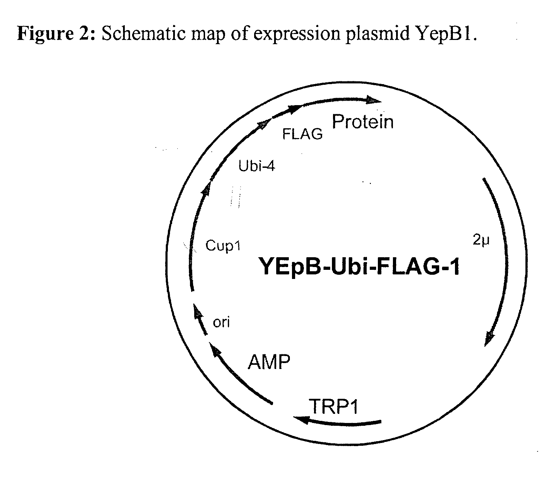

A map of the vector is shown in Figure 2.

### Landmarks

**Table 2:**

| List of resulting GFP fusion proteins. | | |
|---|---|---|
| Landmark | pI | MW (Da) |
| GFP-EIF5a | 5,23 | 43,851.39 |
| GFP-Hsp90beta | 5,08 | 110,031.4 |
| GFP-FKBP25 | 7,26 | 51,913.92 |
| GFP-Lysozym | 7,57 | 43,274.1 |
| BFP-EIF5a | 5,23 | 43,851.39 |
| BFP-Hsp90beta | 5,08 | 110,031.4 |

### Cloning of Landmarks/GFP/BFP-Fusionproteins

The respective cDNAs were amplified by polymerase chain reaction (PCR) from plasmids containing the corresponding cDNAs and digested with the respective endonucleases. Additionally a sequence coding for GFP/BFP respectively was inserted at the N-terminus of the fusion partner, resulting in the corresponding recombinant FLAG-tag-GFP/BFP-fusion plasmid. Ligation reactions were performed in a molar ratio of vector to insert 1:6 and transformed into E.coli HB-101 cells (MAX Efficiency HB-101 cometent cells, GIBCO-BRL Life technologies, Paisley, UK). Positive clones were screened by PCR and restriction analysis, respectively.

### Transformation of yeast strain BJ3505

500 µl of yeast cell suspension were mixed with 500 gl of yeast transfomation buffer (0.1 M lithium acetate, 10 mM TrisHCl, 1 mM EDTA, pH 7.4, sterile filtrated). After centrifugation supernatant was removed. 10 µl of recombinant plasmid DNA (500 ng-1 µg) and 200 µl of PEG solution (1 g polyethylene glycol 4000 dissolved in 1 ml yeast transformation buffer, sterile filtered) were added to the cells and incubated over night at room temperature. The suspension was centrifuged and the supernatant was removed. After a washing step with 1 ml of sterile water, 800 µl of the supernatant were removed and the remaining cells were plated on SCM-Agar-TRP and incubated for 2 days at 30 °C.

### Intracellular protein expression

Transformants were grown on yeast peptone high stability medium (YPHSM, Difco, Augsburg, Germany) at 30 °C. Protein expression was induced by the addition of 10 mM CuSO₄ at an OD₆₀₀ = 0.5. Eight hours post induction cells were harvested, since a maximum level of expression product could be obtained at that time. Prior to further protein analysis, cells had to be ruptured. 1 ml of lysis buffer (50 mM TrisHCl, 150 mM NaCl, 2 mM DTT, 10 % glycerol, pH 7.5) supplemented with a protease inhibitor cocktail (Sigma, St. Louis, MO, USA) was added to the cells. Cells were disrupted in Eppendorf tubes using glass beads (diameter 0.25-0.50 mm) by vortexing 3 times 45 seconds on a IKA vortexter intermittent by 30 seconds on ice. After centrifugation (14.000 rpm, 10min) supernatants were removed and subjected to SDS-Polyacrylamid gel electrophoresis (PAGE) and Western blot analysis.

### Detection of expressed fusion proteins

SDS-PAGE (4-20%) was carried out according to Leammli (11) in a Protean II (BioRad, Hercules, CA, U.S.A) electrophoresis chamber. To check protein integrity Western blot analysis were carried out (2). Samples were transferred onto PROTRAN nitrocellulose transfer membranes 0.2 µm (Schleicher & Schuell, Düren, Germany) by electroblotting. After blocking of non-specific binding sites (TBS- buffer: 20 mM TrisHCl, 500 mM CaCl₂,2 mM CaCl₂, pH 7.5, containing 3 % skim milk powder) blots were incubated with an anti FLAG MI antibody (10 µg/ml, Eastman Kodak, New Haven, CT, USA) as primary antibody and a goat anti mouse peroxidase conjugate (dilution 1: 1 000) as second antibody. Color development was carried out with 4-chloro-1-naphtol (BioRad, Hercules, CA 9 U.S.A.) and H₂O₂.

In Figure 1 Westem blot analysis of landmarks expressed in yeast are shown. As positive control FLAG-tagged Green fluorescent protein was used. As negative control yeast strain BJ3505 transformed with a plasmid without insert was used.

### Purification of the Fusionproteins

300 ml culture broth was centrifuged and washed once with distilled water. After a second centrifugation step, lysis buffer (10 mM Tris/HC1 pH 7.4, 150 mM NaCl, 10% glycerol 1 % Tween 20, 0.1 M TPCK, 0.1 PMSF) was added to the cell pellet in a three fold excess.

Subsequently cells were lysed in a bead mill for 20 min, 4 °C with 0.25 mm glass beads (1/3 of total volume were glass beads). The suspension was centrifuged and the supernatant was further purified by immuno affinity chromatography. Agarose beads immobilized with anti-FLAG antibody M1 (Kodak) were packed into a HR5/5 column with an inner diameter of 0.5 cm (Amersham-Pharmacia, Uppsala, Sweden). The column was connected to an FPLC system (Amersham-Pharmacia) and operated at 100 cm h⁻¹. UV absorbance at 280 mm was continuously monitored with an UV-1 monitor (Amersham-Pharmacia).

As loading buffer the cell lysis buffer was supplemented with 5 mM CaCl₂. For desorption CaCl₂ was exchanged by 10 mM EDTA.

## Claims

1. A fusion protein having the amino acid sequence of a marker protein and the amino acid sequence of a second protein so selected that it yields a predeterminded mass and/or isoelectric point making the fusion protein suitable to represent a reference protein (landmark) in an analytical method.

2. The fusion protein of claim 1 expressed in S. cerevisiae.

3. The fusion protein of claim 1 or 2 wherein the marker protein is selected from the group consisting of Green Fluorescent Protein (GFP), BFP, and the second protein is selected from the group consisting of EIF5a, Hsp90beta, FKBp25, lysozym.

4. The fusion protein of any of the claims 1 to 3 having the following DNA sequences:
Seq. ID No. 1 to Seq. ID No. 6

5. A method of using at least one of the fusion proteins of any of the claims 1 to 4 as a landmark in a multi-stage process for detection of proteins which discriminates different proteins in terms of size, molecular weight.

6. The method of claim 5 wherein a fusion protein of claim 1 to 4 is mixed as a standard reference into an analytical sample applied to a separation medium, and after separation is detected by an appropriate property such as fluorescence or chemiluminescence, staining or blotting and detection with affinity ligands such as labelled antibodies, enzyme conjugated antibodies.

7. The method of claim 5 and/or 6 wherein two fusion proteins are employed which have different marker proteins and/or different second proteins.

8. The method of any of the claims 5 to 7 wherein electrophoretical methods are employed.

9. An analytical kit comprising at least one fusion protein of one of the claims 1 to 4.
